# EUROPEAN PATENT APPLICATION

(11) **EP 4 738 374 A1**
(43) Date of publication of application: **06.05.2026**
(21) Application number: 24209751.7
(22) Date of filing: 30.10.2024
(51) Int. Cl.: G16H 10/60, G06F 21/62

(54) **METHOD, SYSTEN AND COMPUTER NETWORK FOR IDENTIFYING PERSONAL DATA IN A HEALTHCARE DATA STREAM USING A PRE-TRAINED MACHINE LEARNING MODEL**

(71) Applicant: Roche Diagnostics International AG, 6343 Rotkreuz (CH)
(72) Inventor: DE LUCA, Domenico, 6343 Rotkreuz (CH); TAEYMANS, Bert, 6343 Rotkreuz (CH)
(74) Representative: Mewburn Ellis LLP

(57) **Abstract**

A computer-implemented method of processing healthcare data. The method comprising steps of: receiving a healthcare data stream, the data stream including technical data; screening the received data stream to identify potential inclusions of personal data, using a pre-trained machine learning model, within the received data stream; and upon identifying the potential inclusion of personal data within the received data stream, performing a mitigation action with respect to the potential personal data identified in the received data stream. Wherein the pre-trained machine learning model has been trained only on technical data so as to identify technical data in data streams, and so identify potential personal data as anomalous data within the received data stream.

## Description

### TECHNICAL FIELD

The present invention relates to a method, a system, and a computer network.

### BACKGROUND

In diagnostic healthcare, the extensive processing of technical and personal data used to enhance diagnoses often leads to inadvertent breaches of privacy due to the inclusion of personal information in datasets intended for technical use only. The distinction between personal and technical data is important, yet challenging. This is compounded in fast-paced development environments focused on innovation and minimum viable product deployment, where control mechanisms might be bypassed.

The present invention was arrived at in light of the above considerations.

### SUMMARY

Accordingly, in a first aspect, embodiments of the invention provide a computer-implemented method of processing healthcare data, comprising steps of: receiving a healthcare data stream, the data stream including technical data; screening the received data to identify potential inclusions of personal data, using a pre-trained deep learning model, within the received data stream; and upon identifying the potential inclusion of personal data within the received data stream, performing a mitigation action with respect to the potential personal data identified in the received data stream; wherein the pre-trained machine learning model has been fine-tuned only on technical data to identify potential personal data as anomalous data within the received data stream.

Such a method can advantageously improve the quality of the provided technical data by identifying and mitigating against the inclusion of personal data, which also improves privacy compliance.

A healthcare data stream is data collected from one or more healthcare data sources, for example a laboratory information system, hospital management system, in-vitro diagnostic instrument, point-of-care device, or laboratory middleware. It includes, as technical data, technical values pertaining to the source of data(for example reagent levels in an instrument, etc.) . It may also include personal data, such as name, date of birth, location etc. This personal data may have been removed by the source or by the healthcare data system connected to the source, but in some instances may remain (for example due to failure to identify it). The healthcare data stream may be received from a healthcare data system, which may be external to the entity performing the screening.

By stream of data, it is meant a continuously received data stream comprising individual elements of data. The elements of data may be contained within a message sent from the healthcare data source and so the data stream may be a series of received messages. The screening of the data stream can therefore be performed per element of data (or per message) in that the pre-trained machine learning model may interrogate each element of data individually. In other examples, the messages may be grouped or each message may contain plural elements of data, for example a data record. A data record may comprise a group of data elements, for example a clinical entry with the results of a panel of blood tests, or a parameterised radiographic image.

The pre-trained machine learning model may have an architecture size parameter which is below a predetermined threshold, wherein the predetermined threshold is determined based on an amount of computing resource available. The pre-trained machine learning model may have undergone a portability process after training but before deployment, to reduce the size of the model (e.g., to reduce the number of parameters, depth of the neural network, number of nodes, cache size etc.). The pre-trained machine learning model may have no more than 210 million parameters, 100 million parameters, no more than 90 million parameters, no more than 80 million parameters, no more than 70 million parameters, or no more than 66 million parameters. The amount of computing resource may be an amount of memory, for example RAM, and may be no more than 16 GB, no more than 32 GB, or no more than 64 GB. The pre-trained machine learning model should be configured so as to utilise no more than around 5% of the available RAM (e.g., around 800 MB).

The pre-trained machine learning model may have been trained on a dataset of at least 300,000 and/or no more than 700,000 tagged data points. The training data is technical data which, as mentioned above, encompasses only technical values pertaining to the source of data. For example, a device ID, timestamp value for the message, . An example of entries in the dataset is shown in Annex A.

The step of screening the received data may be performed on an edge device. For example, on an in-vitro diagnostic analyser, a point-of-care device, a laboratory middleware, a laboratory information management system, or a desktop computer.

The method may be performed by a data monitoring component. The data monitoring component may be on the edge device, may be located in a cloud computing environment, or may be located on a computer connected to the edge device via a local area network.

The method may further comprise a step of storing the stream of processed data (for example:
classified, screened, or tagged data). in a database when no potential inclusions of personal data have been identified. The database may be external to the originator of the healthcare data stream (e.g., the healthcare data system). That is, the database may be within a different sphere of control (e.g., on a different computer network and/or separated from the entity screening the data by a firewall and/or controlled by a different entity). The successful screening of the stream of data may be notified back to the source of the data.

The method may utilise protocols for managing the data stream, the processed data, and the training dataset, ensuring data diversity to reduce false positives and outlining measures to prevent accidental generation and/or transmission of personal data. The method may utilise: access control methods and authentication (for example multi-factor authentication); and/or data classification methods to further secure the data. For example:
1. Data classification and Ownership
   1.1 Establish clear data ownership by assigning data stewards for different datasets to oversee data handling, usage, and security policies.
   1.2 Implement data classification by categorizing datasets based on sensitivity and privacy requirements to apply appropriate handling protocols, to ensure that only technical data is included.
2. Access Control and Authentication
   2.1 Enforce strict access control policies to ensure that only authorized personnel have access to technical datasets based on their roles and necessity. 2.2 Utilize Multi-Factor Authentication (MFA) for accessing technical datasets to add an extra layer of security.
3.Data encryption and masking
   3.1 Encrypt data at rest and in transit, using strong encryption standards to protect data from unauthorized access during storage and transmission
4. Data Minimization and Diversity
   4.1 Practice data minimization, collect and store only the data necessary for specific, legitimate purposes to reduce the risk of personal data inclusion
   4.2 Ensure data diversity by incorporating diverse datasets in anomaly detection models to minimise biases and false positives, enhancing model reliability.
5. Incident response and data breach protocols
   5.1 Develop an incident response plan to establish a comprehensive incident response plan detailing actions to be taken in case of a data breach, including notification procedures.
   5.2 Conduct regular security audits, perform periodic security assessment and audits to identify vulnerabilities and ensure compliance with data protection regulations.

The mitigation action may include any one or more of: removing and/or anonymizing the identified personal data; sending an alert to a data management component; and generating synthetic technical data or personal data based on at least the received technical data. Synthesising the technical data or personal data may be performed using a machine-learning model, which may be the same machine-learning model used to screen the received data stream where that machine-learning model has generative capability (e.g., is an LLM). The synthesis may be performed using only the technical data. Alternatively the synthesis of technical data may be performed by a separate machine-learning model, trained to synthesize technical or personal data from input technical or personal data. The generation of synthetic technical data or personal data may include the addition of noise to the data or query results, to prevent identification of any individual's data. The mitigation action may be triggered upon identifying the potential inclusion of non-technical (e.g., personal data) within the received data stream.

The method may include a step, performed before the mitigation action but after a determination of potential personal data, of performing a secondary validation to identify personal data. The secondary validation may include regular expression checking for personal data, detecting anomalies, and/or verifying data integrity. The secondary validation may be performed by a secondary validation module which is separate to the entity performing the screening or it may be performed by the same entity performing the screening.

The data stream may be provided via an API exposure layer, which interfaces with the pre-trained machine learning model for screening.

The method may include an initial validation step performed before screening the receive data, the initial validation step checking the format of the data stream and/or the integrity of the data stream.

The method may further comprise steps of: obtaining feedback on the identification of potential inclusions of personal data; and adjusting the pre-trained machine learning model based on the obtained feedback. For example, a reinforcement learning approach may be applied to the pre-trained machine learning model.

The pre-trained machine learning model may be a large language model. In other examples, the pre-trained machine learning model may be a supervised learning model trained on labelled data sets of technical data or an unsupervised model designed to detect outliers from the technical data on which it was trained. The pre-trained machine learning model may be a transformer based encoder only model. It may be a pre-trained model which has subsequently undergone a fine-tuning process. For example, the model may be a GPT-2, DistilBERT, or RoBERTa based model. The model may be trained to identify out of distribution patterns (i.e. to distinguish patterns that the model has not been trained on) as anomalous data. The model may use gram matrices to characterize activity patterns. Gram matrices as used in anomaly detection can be effectively incorporated during the fine-tuning process of the LLM.

LLMs can adapt to a wide variety of data formats and contexts due to their extensive training on diverse datasets. This adaptability allows them to recognize and flag potential privacy concerns in data that may not follow predictable patterns, surpassing rule-based systems. Further, unlike rule-based systems that operate on explicit patterns, LLMs are able to understand the context around data, allowing them to better distinguish between personal and technical information. This context-aware processing reduces the chance of personal data slipping through due to nuanced language or unconventional data presentation.

Further, by using only technical data for training and leveraging the LLM's capabilities to identify anomalies, the methods and systems disclosed herein can minimise or reduce false positives (where benign data is incorrectly flagged as containing personal data) and false negatives (where personal data is not detected). LLMs are also able to process large volumes of data quickly and efficiently, which makes them suitable for environments with extensive data processing needs. This is particularly useful in the context of healthcare data, where volumes continually grow.

The data stream may be an encrypted data stream, which is decrypted during the screening process and re-encrypted before storage or subsequent retransmission.

In second aspect, embodiments of the present invention provide a data monitoring component, including one or more processors and memory, the memory containing machine executable instructions which, when executed on the processor(s), cause the processor to:
receive a healthcare data stream, the data stream including technical data;
screen the received data stream to identify potential inclusions of personal data, using a pre-trained machine learning model, within the received data stream; and
upon identifying the potential inclusion of personal data within the received data stream, perform a mitigation action with respect to the potential personal data identified in the received data stream;
wherein the pre-trained machine learning model has been trained only on technical data so as to identify technical data in data streams, and so identify potential personal data as anomalous data within the received data stream.

The machine executable instructions may cause the processor to perform the method of the first aspect, including any one, or any combination insofar as they are compatible, of the optional features set out with reference thereto.

In a third aspect, embodiments of the invention provide a computer network including:
the data monitoring component of the second aspect;
a healthcare data source, connected to the data monitoring component and which provides the healthcare data stream to the data monitoring component; and
a database;
wherein the data monitoring component is configured to transmit the stream of data to the database when no potential inclusions of personal data identified.

The computer network may further comprise a synthetic data generator, which receives at least a part of the data stream from the data monitoring component, and is configured to generate synthetic technical data based on the received at least a part of the data stream.

The computer network may further comprise a secondary validation module, connected to the data monitoring component, and configured to perform a secondary validation to identify personal data.

The invention includes the combination of the aspects and preferred features described except where such a combination is clearly impermissible or expressly avoided.

Further aspects of the present invention provide: a computer program comprising code which, when run on a computer, causes the computer to perform the method of the first aspect; a non-transitory computer readable medium storing a computer program comprising code which, when run on a computer, causes the computer to perform the method of the first aspect; and a computer system programmed to perform the method of the first aspect.

### BRIEF DESCRIPTION OF THE DRAWINGS

Fig. 1A shows a method;
Fig. 1B shows an architecture of a pre-trained machine learning model;
Fig. 2 shows a system;
Fig. 3 shows a swim-lane diagram of a continuous anomaly detection process;
Fig. 4 shows a swim-lane diagram of a real-time integration with external validation algorithms process; and
Fig. 5 shows a swim-lane diagram of a whole system process.

### DETAILED DESCRIPTION

Aspects and embodiments of the present invention will now be discussed with reference to the accompanying figures. Further aspects and embodiments will be apparent to those skilled in the art.

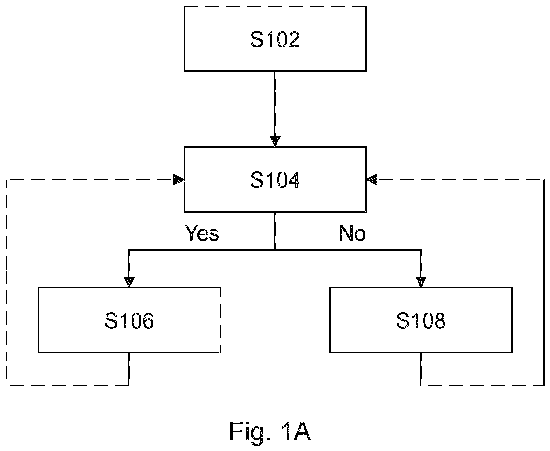

Fig. 1A shows a method. In a first step of the method, S102, a healthcare data stream is received, the data stream including technical data. Next, in step S104, the healthcare data stream is screened for potential inclusions of personal data, using a pre-trained deep learning model. If personal data is identified, 'Yes', the method moves to step S106 where a mitigation action is performed with respect to the potential personal data identified in the received data stream. If no personal data is identified, the method moves to step S108 where the stream of processed data is stored in a database.

As has been discussed previously, the data stream can take the form of a stream of messages each containing one or more data elements. The screening is performed either on the data elements individually or in groups, or per data record (where the message contains a data record). After each element, or group of elements, have been screened, the method returns to step S104 when the next data element(s) is/are screened.

In this example, the pre-trained machine learning model is a large language model of the type discussed previously. In this example, it was an implementation of the DistilBERT transformer model. The model is a lightweight, efficient version of BERT (Bidirectional Encoder Representations from Transformers) that retains 97% of BERT's performance whilst being 60% faster and 40% smaller. It has 66 million parameters, and was fine-tuned using only technical data (and requiring a smaller volume of data than for the original pre-training). Fine-tuning typically only needs a smaller dataset that is representative of the specific domain. The training data was high-quality and well-labelled. The training data provided had between 100,000 and 2,000,000 tagged data points, preferably between 300,000 and 700,000 tagged data points. An example of the training data is provided in Annex A.

Screening is achieved by providing the specific data element to the model, and identify whether the element is out-of-distribution (i.e., represents a pattern or element that the model was not trained on). The Gram Matrices anomaly detection technique was used, as it can be added to the standard architecture. This involved fine-tuning the model to recognise normal patterns and flagging deviations from this as potential out-of-distribution instances. When technical data is incorrectly flagged as out-of-distribution (i.e., a false positive) the model can be fine-tuned again and the Gram matrices updated quickly.

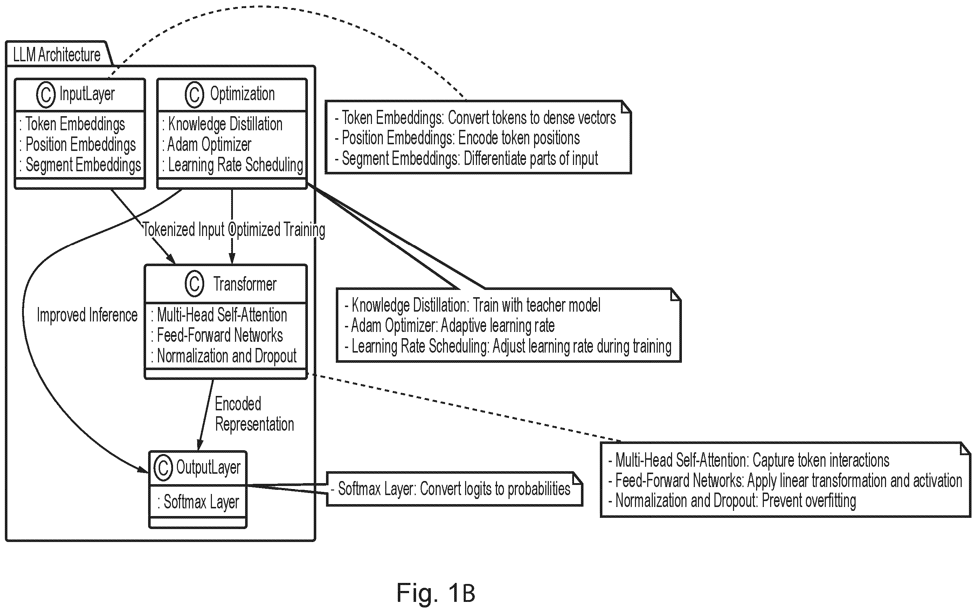

The model was trained to provide classification similar to sentiment analysis, and so typically only suitable to be extended to the specific recognition task at hand. For this reason, the model was a transformer based "encoder only" model with an architecture as shown in Fig. 1B which includes multiple transformer layers. The use of Gram matrices here allows the internal activity patterns of the model to be captured, which facilitates the out-of-distribution detection in samples.

The mitigation action in S106 can take a number of forms. In some examples, the data identified as being anomalous is simply discarded / deleted and therefore not stored. In other examples, it may be anonymized. In such examples, the method may include replacing the personal data with synthetic non-identifying personal data (e.g., a synthetic name is generated to replace the name in the data stream). The mitigation action can also include, or be only, the sending of an alert to a data management component (which can be the source of the data) and the halt of the data stream. The alert can also be sent when the data is simply discarded (as a note to the data source). Further, the mitigation action can include the synthesis of replacement (technical or personal) data such that the data stream is contiguous and continuous.

Other examples of the method include an initial validation step, performed before step S104 but after step S102, of checking the format of the data stream and/or the integrity stream. For example, by comparing the elements of the data stream to a specification of the data, or computing checksums for the data stream or elements thereof to check integrity. Some examples of the method also include, after step S106 or S108, obtaining feedback on the identification of potential inclusions of personal data, and adjusting or fine-tuning the LLM based on the obtained feedback. In some examples, the data stream is received in encrypted form, and so there may be step after step S102 of decrypting the data stream or elements thereof, and a step after S104 and before S108 of re-encrypting the data stream or elements therefore before sending for storage.

An example of a received message is shown below:

```
 {
  "device_id": "DX10023",
  "timestamp" : "2024-09-23T12:00:00Z",
  "heart_rate": 75,
  "patient_name": "John Doe",
  "patient_age": 45,
  "patient_id": "P12345",
  "test_result": "Negative"
  }
```

As can be seen, the received message includes two entries (device_id and timestamp) which are technical data. The following entries, heart_rate, patient_name, patient_age, patient_id, and test_result are not considered technical data, and so would be screened. The mitigation action in this instance replaces the values of the personal data entries with a redacted flag:

```
 {
  "device_id": "DX10023",
  "timestamp" : "2024-09-23T12:00:00Z",
  "heart_rate": [REDACTED],
  "patient_name": [REDACTED],
  "patient_age": [REDACTED],
  "patient_id": [REDACTED],
  "test_result": [REDACTED]
  }
```

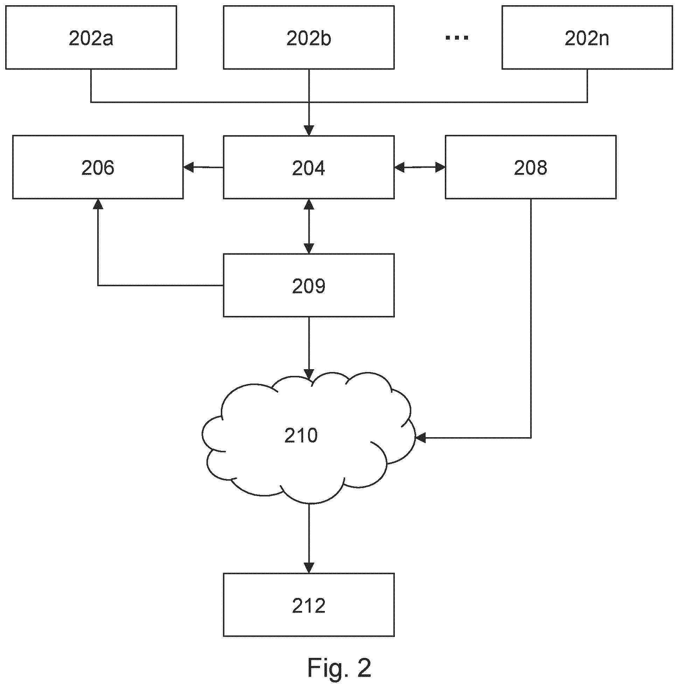

Fig. 2 shows a system. The system includes one or more sources of data 202a - 202n, for example in-vitro diagnostic instruments, point of care devices, and hospital / laboratory information systems. These provide stream(s) of data to a data monitoring component 204, which is configured to perform the method in Fig. 1. Where the data monitoring component clears the data, i.e., gets to step S108, it transmits the data through wide area network 210 to a data store 212. The network also includes a data management component 206, and data synthesiser 208 to which the data monitoring component is connected.

When the data monitoring component 204 issues an alert that possible personal data was identified, it does so by sending a message to the data management component 206. In some examples, the data management component may be a given source of data 202a - 202n. Indeed, some examples, the data monitoring component 204 may be located in or installed on a given source of data (e.g., in an IVD instrument or point of care device). In these examples, as has been discussed above, a pre-trained machine learning model of reduced complexity and/or with reduced resource requirements may be used to ensure it can effectively screen data before it is transmitted off of the device for sharing.

The data monitoring component 208 is also connected to a data synthesiser 208, which can use a generative model to produce data to replace the data flagged as possibly containing personal data. This produced data can take two forms: (i) replacement technical data, which fits the technical data around it (so that the data stream is contiguous and continues); or (ii) replacement personal data, where the replacement personal data is generated so as fulfil the requirements of the data whilst not disclosing any personally identifiable information (for example, the replacement of a name with a replacement, generated, name).

The network also includes a secondary validation module 209 which is connected to the data monitoring component 204 and either receives a processed data stream from the data monitoring component or flagged potential personal data. The secondary validation module then performs a secondary check of the received elements of data (either in the stream, or the specifically flagged potential personal data). For example, the secondary validation module may apply regular expression checking to screen for dates of birth, postal addresses, and/or email addresses.

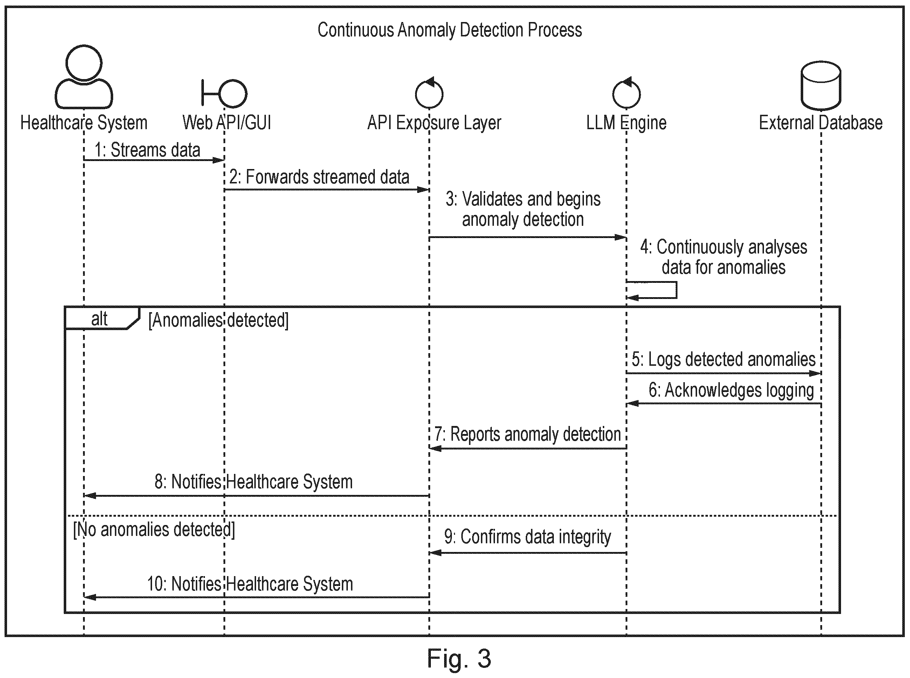

The system architecture can be implemented at a container level visualisable using a C4 framework. The components shown in Figs. 3 - 5 are briefly discussed:
Web API/GUI - can be implemented as an external system, and provides a graphical user interface for healthcare providers to interact with the LLM engine, particularly for submitting datasets and retrieving processing results, including those related to reinforcement from human feedback (RLHF);
API Exposure layer - acts as a gateway to the LLM engine, handling requests from the Web API/GUI and other external systems. It is responsible for exposing the LLM engine's capabilities, including data analysis and integration with other components;
LLM engine - the core component where anomaly detection, synthetic data generation/refinement, and RLHF are conducted. It is integrated directly with the API exposure layer to process data and interact with the external database for data storage needs;
Database - classified as an external system, it stores logs, configurations, and temporary data utilised for the LLM engine's operations. The database external status highlights the role as a shared or third party service that can be accessed by the LLM engine. It can be an SQL or NoSQL database;
Synthetic Data Generator and Validation Algorithms - external systems that interact with the API exposure layer for synthetic data generation and additional data validation, respectively.

Fig. 3 shows a swim-lane diagram of a continuous anomaly detection process. As shown, a healthcare system streams data to a web API/GUI, which forwards this stream on to an API exposure layer. The API exposure layer can perform initial validation of the type discussed previously, before using the LLM engine to begin screening. The LLM engine continuously analyses the data for anomalies, and where it detects one (box alt [Anomalies detected]) it logs the detected anomalies. In this example, the anomalies are logged in an external database, but they may be logged on the healthcare system instead. Once the logging of anomalies has been acknowledged, the LLM engine reports the anomaly detection to the API exposure layer which in turn notifies the healthcare system. If the LLM does not detect an anomaly ([No anomalies detected]) the LLM engine will instead confirm the data integrity to the API exposure layer which, in turn, notifies the healthcare system.

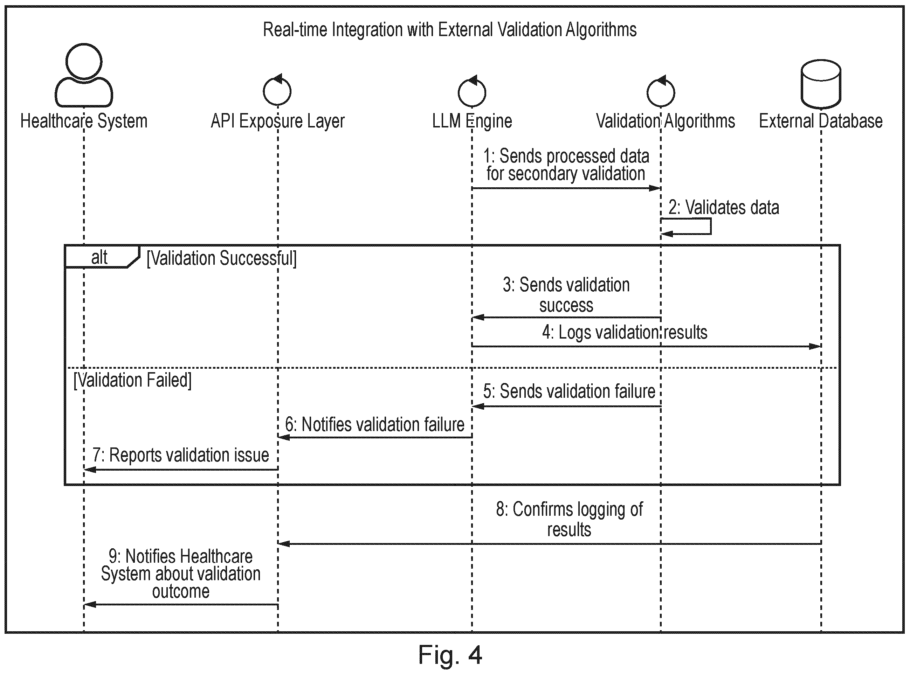

Fig. 4 shows a swim-lane diagram of a real-time integration with external validation algorithms process. This diagram begins after the LLM has received and screened the data stream, and when the data stream has been cleared it is sent to a validation algorithms container for secondary validation (or, when potential anomalies are identified, these are forwarded to the container for a secondary check before reporting). The secondary validation is performed, and when successful ([Validation Successful]) this is indicated back to the LLM engine which in turn logs the validation results (here in an external database). Where the secondary validation is unsuccessful ([Validation failed]), the validation algorithms container sends a validation failure message to the LLM engine which notifies this to the API exposure layer. This is then reported to the healthcare system. In either case, the external database confirms the logging of the results to the API exposure layer which in turn notifies the healthcare system about the validation outcome.

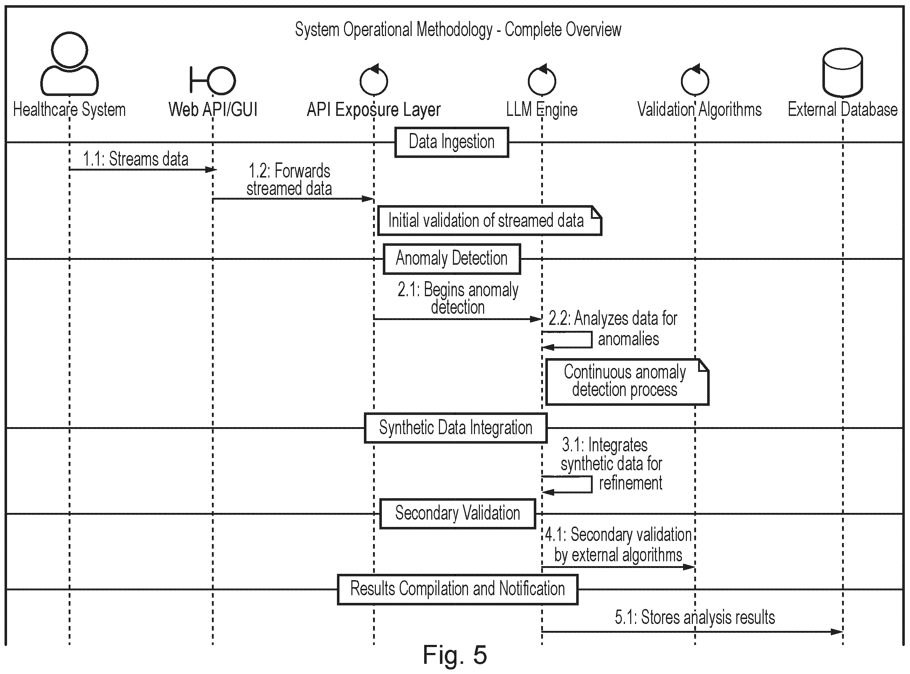

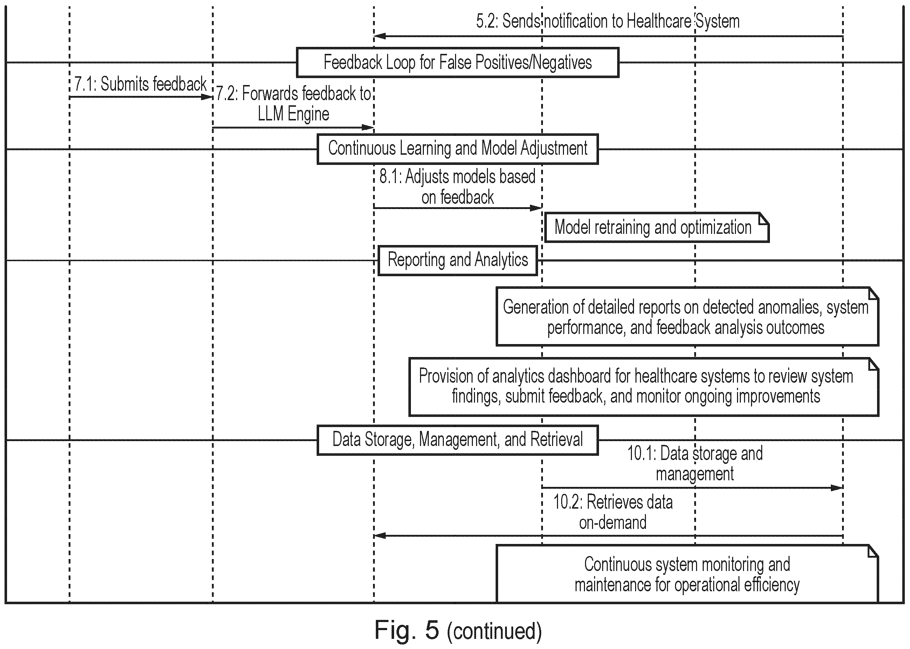

Fig. 5 shows a swim-lane diagram of a whole system process. As with previous discussions, it begins with the healthcare system streaming data to a web API/GUI which in turn forwards that streamed data to an API exposure layer. Here an initial validation of the streamed data can be performed of the kind discussed previously. These steps can, collectively, be referred to as data ingestion. Next, an anomaly detection section starts, which begins with the API exposure layer forwarding the data stream to the LLM engine to begin anomaly detection. The LLM engine then analyses the data for anomalies in a continuous anomaly detection process.

This process includes a synthetic data integration section, whereby the LLM integrates synthetically generated data for refinement of the data stream and/or the LLM itself. Next, the process enters a secondary validation section whereby the LLM sends the screened data stream or specifically flagged data elements considered to be anomalous to a validation algorithms container which performs secondary validation as discussed previously. When the data stream is cleared the results of the analysis are then stored in an external database and the external database sends a notification to the healthcare system via the API exposure layer. This takes place in the results compilation and notification section.

The process also includes a Feedback Loop for False Positives/Negative section, where the healthcare system (e.g., an operator thereof) provides feedback to the web API/GUI which in turn forwards this to the API exposure layer. The API exposure layer, in a Continuously Learning and Model Adjustment section, adjusts the model(s) in the LLM engine based on the feedback resulting in model retraining and optimization. In a Reporting and Analytics section, detailed reports on the generated anomalies, system performance, and feedback analysis outcomes can be generated. There can also be provision of an analytics dashboard for the healthcare system to review system findings, provide feedback, and monitor ongoing improvements.

Finally, the process includes a Data Storage, Management, and Retrieval section where the LLM engine causes the screened data to be stored and managed on the external database. This data can be retrieved on-demand by the API exposure layer (e.g., in response to a request from the healthcare system) which allows for continuous system monitoring and maintenance for operational efficiency.

The systems and methods of the above embodiments may be implemented in a computer system (in particular in computer hardware or in computer software) in addition to the structural components and user interactions described.

The term "computer system" includes the hardware, software and data storage devices for embodying a system or carrying out a method according to the above described embodiments. For example, a computer system may comprise a central processing unit (CPU), input means, output means and data storage. The computer system may have a monitor to provide a visual output display. The data storage may comprise RAM, disk drives or other computer readable media. The computer system may include a plurality of computing devices connected by a network and able to communicate with each other over that network.

The methods of the above embodiments may be provided as computer programs or as computer program products or computer readable media carrying a computer program which is arranged, when run on a computer, to perform the method(s) described above.

The term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.

While the disclosure has been described in conjunction with the exemplary embodiments described above, many equivalent modifications and variations will be apparent to those skilled in the art when given this disclosure. Accordingly, the exemplary embodiments of the disclosure set forth above are considered to be illustrative and not limiting. Various changes to the described embodiments may be made without departing from the spirit and scope of the disclosure.

In particular, although the methods of the above embodiments have been described as being implemented on the systems of the embodiments described, the methods and systems of the present disclosure need not be implemented in conjunction with each other, but can be implemented on alternative systems or using alternative methods respectively.

The features disclosed in the description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the disclosure in diverse forms thereof.

While the disclosure has been described in conjunction with the exemplary embodiments described above, many equivalent modifications and variations will be apparent to those skilled in the art when given this disclosure. Accordingly, the exemplary embodiments of the disclosure set forth above are considered to be illustrative and not limiting. Various changes to the described embodiments may be made without departing from the spirit and scope of the disclosure.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

```
 Annex A
 {
  "specversion": "1.0",
  "type": "com.healthcare.device.event",
  "source": "/devices/{deviceld}",
  "id": "{uniqueEventId}",
  "time": "{eventTimestamp}",
  "subject": "device.{deviceType}",
  "datacontenttype": "application/json",
  "data": {
     "deviceId": "{deviceId}",
     "deviceType": "{deviceType}",
     "manufacturer": "{manufacturerName}",
     "modelNumber": "{modelNumber}",
     "serialNumber": "{serialNumber}",
     "firmwareVersion": "{version}",
     "softwareversion" : "{version}",
     "hardwareRevision": "{revision}",
     "eventCode": "{eventCode}",
     "eventDescription": "{eventDescription}",
     "eventSeverity": "{severityLevel}", // e.g., "info", "warning", "critical"
     "operationalStatus": "{operationalStatus}", // e.g., "operational",
     "maintenance", "offline"
     "measurements": {
       "temperature": "{value}",
       "pressure": "{value}",
       "flowRate": "{value}",
       "signalQuality" : "{value}",
       "batteryLevel": "{percentage}",
       "powerConsumption": "{watts}"
       // Additional measurements relevant to diagnostic devices
     },
     "calibration": {
       "lastCalibrationDate": "{timestamp}",
       "nextCalibrationDue": "{timestamp}",
       "calibrationStatus": "{status}",
       "calibratedBy": "{technicianId}"
     },
     "maintenance": {
       "lastMaintenanceDate": "{timestamp}",
       "nextMaintenanceDue": "{timestamp}",
       "maintenanceStatus": "{status}",
       "maintenancePerformedBy": "{technicianId}",
       "maintenanceLogs": [
          {
            "maintenanceId": "{maintenanceId}",
            "description": "{description}",
            "actionsTaken": "{actions}",
            "partsReplaced": [
               {
                 "partId": "{partId}",
                 "partName": "{partName}",
                 "serialNumber": "{serialNumber}"
               }
               // Additional parts
            ]
          }
          // Additional maintenance entries
       ]
     },
     "alerts": [
       {
          "alertId": "{alertId}",
          "alertCode": "{alertCode}",
          "alertType": "{alertType}",
          "alertMessage": "{alertMessage}",
          "timestamp": "{timestamp}",
          "resolved": "{boolean}",
          "resolution": {
            "resolvedBy": "{technicianId}",
            "resolutionTime": "{timestamp}",
            "resolutionDescription": "{description}"
          }
       }
       // Additional alert entries
     ],
     "usageStatistics": {
       "totalOperatingHours": "{hours}",
       "usageCount": "{numberOfUses}",
       "averageUsageDuration": "{duration}",
       "lastUsed": "{timestamp}"
       // Additional usage statistics
     },
     "connectivity" : {
       "networkstatus": "{status}",
       "ipAddress": "{ipAddress}",
       "macAddress": "{macAddress}",
       "signalStrength": "{value}",
       "connectionType": "{type}", // e.g., "WiFi", "Ethernet", "Cellular"
       "latency": "{milliseconds}"
     },
     "location": {
       "facility": "{facilityName}",
       "department": "{departmentName}",
       "room": "{roomNumber}",
       "coordinates": {
          "latitude": "{latitude}",
          "longitude": "{longitude}"
       }
     },
     "environmentalConditions": {
       "ambientTemperature": "{value}",
       "humidity": "{percentage}",
       "vibration": "{value}",
       "noiseLevel": "{decibels}"
     },
     "softwareModules": [
       {
          "moduleld": "{moduleId}",
          "moduleName": "{moduleName}",
          "version": "{version}",
          "status": "{status}",
          "lastUpdate": "{timestamp}"
       }
       // Additional software modules
     ],
     "timestamps": {
       "eventGenerated": "{timestamp}",
       "eventReceived": "{timestamp}",
       "lastUpdated": "{timestamp}"
       // Additional relevant timestamps
     },
     "compliance": {
       "regulatoryStandards": [
          "ISO 13485",
          "FDA 21 CFR Part 820"
          // Additional standards
       ],
       "complianceStatus": "{status}", // e.g., "compliant", "non-compliant"
       "lastAudit": "{timestamp}",
       "auditFindings": [
          {
            "findingId": "{findingId}",
            "description": "{description}",
            "status": "{status}", // e.g., "open", "closed"
            "correctiveActions": "{actions}"
          }
          // Additional findings
       ]
     },
     "metadata": {
       "tags": [
          "{tag1}",
          "{tag2}"
          // Additional tags
       ],
       "notes": "{additionalNotes}",
       "documentationLinks": [
          "{url1}",
          "{url2}"
          // Additional links
       ]
     }
  }
  }
```

## Claims

1. A computer-implemented method of processing healthcare data, comprising steps of:
receiving a healthcare data stream, the data stream including technical data;
screening the received data stream to identify potential inclusions of personal data, using a pre-trained machine learning model, within the received data stream; and
upon identifying the potential inclusion of personal data within the received data stream, performing a mitigation action with respect to the potential personal data identified in the received data stream;
wherein the pre-trained machine learning model has been trained only on technical data so as to identify technical data in data streams, and so identify potential personal data as anomalous data within the received data stream.

2. The computer-implemented method of claim 1, wherein the pre-trained machine learning model has an architecture size parameter which is below a predetermined threshold, wherein the predetermined threshold is determined based on an amount of computing resource available.

3. The computer-implemented method of claim 1 or claim 2, the method further comprising a step of storing the stream of processed data in a database when no potential inclusions of personal data have been identified.

4. The computer-implemented method of any preceding claim, wherein the mitigation action includes any one or more of:
removing and/or anonymizing any identified personal data;
sending an alert to a data management component; and
generating synthetic technical data based on at least the received technical data.

5. The computer-implemented method of any preceding claim, further comprising a step, performed before the mitigation action, of performing a secondary validation to identify personal data.

6. The computer-implemented method of claim 5, wherein the secondary validation includes regular expression checking for personal data, detecting anomalies, and/or verifying data integrity.

7. The computer-implemented method of any preceding claim, wherein the data stream is provided via an API exposure layer, which interfaces with the pre-trained machine learning model for screening.

8. The computer-implemented method of any preceding claim, including an initial validation step performed before screening the received data, the initial validation step checking the format of the data stream and/or the integrity of the data stream.

9. The computer-implemented method of any preceding claim, further comprising steps of obtaining feedback on the identification of potential inclusions of personal data; and adjusting the pre-trained machine learning model based on the obtained feedback.

10. The computer-implemented method of any preceding claim, wherein the pre-trained machine learning model is a large language model.

11. The computer-implemented method of any preceding claim, wherein the data stream is an encrypted data stream, which is decrypted during the screening process and re-encrypted before storage or subsequent retransmission.

12. A data monitoring component, including one or more processors and memory, the memory containing machine executable instructions which, when executed on the processor(s), cause the processor to:
receive a healthcare data stream, the data stream including technical data;
screen the received data stream to identify potential inclusions of personal data, using a pre-trained machine learning model, within the received data stream; and
upon identifying the potential inclusion of personal data within the received data stream, perform a mitigation action with respect to the potential personal data identified in the received data stream;
wherein the pre-trained machine learning model has been trained only on technical data so as to identify technical data in data streams, and so identify potential personal data as anomalous data within the received data stream.

13. A computer network including:
the data monitoring component of claim 12;
a healthcare data source, connected to the data monitoring component and which provides the healthcare data stream to the data monitoring component; and
a database;
wherein the data monitoring component is configured to transmit the stream of data to the database when no potential inclusions of personal data identified.

14. The computer network of claim 13 further comprising:
a synthetic data generator, which receives at least a part of the data stream from the data monitoring component, and is configured to generate synthetic technical data based on the received at least a part of the data stream.

15. The computer network of claim 13 or 14, further comprising a secondary validation module, connected to the data monitoring component, and configured to perform a secondary validation to identify personal data.
